(19) Europäisches Patentamt
European Patent Office
Office européen des brevets

(11) EP 0 479 822 B1

(12) EUROPEAN PATENT SPECIFICATION

(45) Date of publication and mention of the grant of the patent: **18.08.1999 Bulletin 1999/33**

(21) Application number: **90909151.4**

(22) Date of filing: **26.06.1990**

(51) Int Cl.[6]: **C07D 473/00**, A61K 31/52

(86) International application number: **PCT/GB90/00984**

(87) International publication number: **WO 91/00282 (10.01.1991 Gazette 1991/02)**

# (54) THERAPEUTIC NUCLEOSIDES

THERAPEUTISCHE NUKLEOSIDE

NUCLEOSIDES THERAPEUTIQUES

(84) Designated Contracting States:
**AT BE CH DE DK ES FR GB IT LI LU NL SE**

(30) Priority: **27.06.1989 US 371789**

(43) Date of publication of application:
**15.04.1992 Bulletin 1992/16**

(73) Proprietor: **THE WELLCOME FOUNDATION LIMITED**
**Greenford, Middlesex UB6 0NN (GB)**

(72) Inventor: **DALUGE, Susan, Mary**
**Chapel Hill, NC 27514 (US)**

(74) Representative: **Garrett, Michael et al**
**Glaxo Wellcome plc,**
**Glaxo Wellcome House,**
**Berkeley Avenue**
**Greenford, Middlesex UB6 0NN (GB)**

(56) References cited:
**EP-A- 349 242** **FR-A- 2 626 002**

- **CHEMICAL ABSTRACTS, Vol. 108, No. 21, 23 May 1988, (Columbus, Ohio, US), V.E. MARQUEZ et al.: "Synthesis of 2',3'-Dideoxycyclopentenyl Carbocyclic Nucleosides as Potential Drugs for the Treatment of AIDS", see page 749* Abstract 187147s, & Nucleosides Nucleotides 1987, 6(1-2), 239-44***
- **CHEMICAL ABSTRACTS, Vol. 110, No. 7, 13 February 1989, (Columbus, Ohio, US), R.VINCE et al.: "Potent and Selective Activity of a New Carbocyclic Nucleoside Analog (Carbovir: NSC 614846) against Human Immunodeficiency Virus in Vitro", see pages 15-16* Abstract 50717z, & Biochem. Biophys. Res. Commun. 1988, 156(2), 1046-53***
- **CHEMICAL ABSTRACTS, Vol. 111, No. 5, 31 July 1989, (Columbus, Ohio, US), see page 650* Abstract 39839r, & JP, A, 01 22 853 (Asahi Galss Co., Ltd) 25 January 1989***

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 479 822 B1

## Description

The present invention relates to purine nucleoside analogues containing an unsaturated carbocyclic ring in place of the sugar residue, pharmaceutically acceptable derivatives thereof, and their use in therapy, particularly for the treatment or prophylaxis of certain viral infections.

AIDS is an immunosuppressive or immunodestructive disease that predisposes subjects to fatal opportunistic infections. Characteristically, AIDS is associated with a progressive depletion of T-cells, especially the helper-inducer subset bearing the $OKT^4$ surface marker.

Human Immunodeficiency Virus (HIV) has been reproducibly isolated from patients with AIDS or with the symptoms that frequently precede AIDS. HIV is cytopathic and appears to preferentially infect and destroy T-cells bearing the $OKT^4$ marker , and it is now generally recognized that HIV is the etiological agent of AIDS.

Since the discovery that HIV is the etiological agent of AIDS, numerous proposals have been made for anti-HIV chemotherapeutic agents that may be effective in treating AIDS sufferers. Thus, for example, Europea. Patent Specification No, 196185 describes 3'-azido-3'-deoxythymidine (which has the approved name zidovudine), its pharmaceutically acceptable derivatives and their use in the treatment of human retrovirus infections including AIDS and associated clinical conditions. Vince et al., Antiviral Research, 9 (1/2), 120 (1988) describes certain carbocyclic purine nucleosides and their use against HIV. At the Second International Conference on antiviral research Williamsburg VA, 10/14 April 1988, (±)-9-(cis-4-(hydroxymethyl)-2-cyclopentenyl) guanine (NSC - 614846), also known as carbovir, was disclosed.

World wide, hepatitis 8 (HBV) is a viral pathogen of major consequence. It is most common in Asian countries, and prevalent in sub-Saharan Africa. The virus is aetiologically associated with primary hepatocellular carcinoma and is thought to cause 80% of the world's liver cancer. In the United States more than ten thousand people are hospitalised for HBV illness each year, an average of 250 die with fulminant disease. The United States currently contains an estimated pool of 500,000-1-million infectious carriers. Chronic active hepatitis will develop in over 25% of carriers, and often progresses to cirrhosis. It is estimated that 5000 people die from HBV related cirrhosis each year in the USA, and that perhaps 1000 die from HBV-related liver cancer. Even when a universal HBV vaccine is in place, the need for effective anti-HBV compounds will continue. The large reservoir of persistently infected carriers, estimated at 220 million worldwide, will receive no benefit from vaccination and will continue at high risk for HBV induced liver disease. This carrier population serves as the source of infection of susceptible individuals perpetuating the instance of disease particularly in endemic areas or high risk groups such as IV drug abusers and homosexuals. Thus, there is a great need for effective antiviral agents, both to control the chronic infection and reduce progression to hepatocellular carcinoma.

Clinical effects of infection with the HBV virus range from headache, fever, malaise, nausea, vomiting, anorexia and abdominal pains. Replication of the virus is usually controlled by the immune response, with a course of recovery lasting weeks or months in humans, but infection may be more severe leading to persistent chronic liver disease as outlined above. In "Viral Infections of Humans" (second edition, Ed., Evans, A.S. (1982) Plenum Publishing Corporation, New York), Chapter 12 describes in some detail, the aetiology of viral hepatitis infections.

Hepatitis B virus (HBV) is a small DNA containing virus which infects humans. It is a member of the class of closely related viruses known as the hepadnaviruses, each member of which selectively infects either mammalian or avian hosts, such as the woodchuck and the duck. Recent insights into the mechanism of replication of the hepadnavirus genome by reverse transcription of an RNA intermediate, suggests that this reverse transcriptase is a logical chemotherapeutic target.

JP 64-22853 discloses a series of antiviral purine and pyridine nucleoside analogues subsituted in the 9 position by cyclopent-2-ene-4-methanol EP 0349242 disclose a series of antiviral purine nucleoside analogues substituted in the 9 position by cyclopentene-4-methanol.

It has now been discovered that certain purine nucleoside analogues containing an unsaturated carbocyclic ring as referred to below, are useful for the treatment or prophylaxis of viral infections for example hepatitis B infections, and retroviral infections, especially AIDS.

According to a feature of the present invention, novel compounds of the formula (I) are provided:

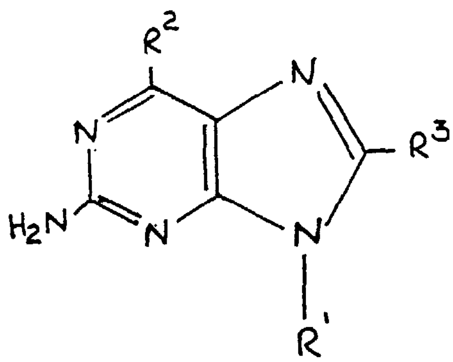

(I)

wherein $R^1$ represents

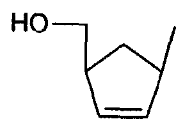

(A)

$R^2$ represents (2-cyclohexen-1-yl)thio, (2-cyclopenten-1-yl)amino, (8-aminooctyl)amino or (cyclopropylmethyl)amino; and $R^3$ is hydrogen

The most preferred isomers are those in which the hydroxymethyl group is cis to the purine in compounds of formula (I). It is to be understood that the present invention encompasses the individual enantiomers of the compounds of formula (I) as well as wholly or partially racemic mixtures of such enantiomers even though the precise structures as drawn relate to one enantiomer.

The compounds of formula (I) above are hereinafter referred to as the compounds according to the invention.

In one aspect of the invention there are provided the compounds according to the invention for use in medical therapy particularly for the treatment or prophylaxis of retroviral infections and heptatis B infections.

Examples of retroviral infections which may be treated or prevented in accordance with the invention include human retroviral infections such as Human Immunodeficiency Virus (HIV), HIV-1 or HIV-2 and Human T-cell Lymphotropic Virus (TL) e.g. HTLV-I or HTLV-II infections. The compounds according to the invention are especially useful for the treatment or prophylaxis of AIDS and related clincial conditions such as AIDS-related complex (ARC), progressive generalised lymphadenopathy (PGL), AIDS-related neurological conditions, such as multiple sclerosis or tropical paraparesis, anti-HIV antibody-positive and HIV-positive conditions, Kaposi's sarcoma and thrombocytopenia purpura. The compounds may also be used in the treatment or prevention of psoriasis.

The compounds of the present invention are particularly applicable for the treatment of asymptomatic infections or diseases in humans caused by or associated with human retroviruses.

In a further aspect of the present invention there is included:-

a) A method for the treatment or prophylaxis of retroviral infections and hepatitis B infections which comprises treating the subject with a therapeutically effective amount of a compound according to the invention.

b) Use of a compound according to the invention in the manufacture of a medicament for the treatment or prophylaxis of any of the above-mentioned infections or conditions.

Preferred esters of the compounds of the invention include carboxylic acid esters in which the non-carbonyl moiety of the ester grouping is selected from straight or branched chain alkyl e.g. n-propyl, t-butyl, n-butyl, alkoxyalkyl (e.g. methoxymethyl), aralkyl (e.g. benzyl), aryloxyalkyl (e.g. phenoxymethyl), aryl (e.g. phenyl optionally substituted by halogen, $C_{1-4}$ alkyl or $C_{1-4}$ alkoxy); sulphonate esters such as alkyl- or aralkylsulphonyl (e.g. methanesulphonyl); amino acid esters (e.g. L-valyl or L-isoleucyl); and mono-, di- or tri-phosphate esters. The phosphate esters may be further esterified by, for example, a $C_{1-20}$ alcohol or a reactive derivative thereof, or by a 2,3-di($C_{6-24}$)arylglycerol.

With regard to the above-described esters, unless otherwise specified, any alkyl moiety present advantageously contains 1 to 18 carbon atoms, particularly 1 to 4 carbon atoms. Any aryl moiety present in such esters advantageously comprises a phenyl group.

Any reference to any of the above compounds also includes a reference to a pharmaceutically acceptable salt thereof.

Examples of pharmaceutically acceptable salts of the compounds according to the invention and pharmaceutically acceptable derivatives thereof include base salts, eg derived from an appropriate base, such as alkali metal (e.g. sodium), alkaline earth metal (e.g. magnesium) salts, ammonium and $NW_4^+$ (wherein W is $C_{1-4}$ alkyl). Physiologically acceptable salts of a hydrogen atom or an amino group include salts of organic carboxylic acids such as acetic, lactic, tartaric, malic, isethionic, lactobionic and succinic acids; organic sulfonic acids such as methanesulfonic, ethanesulfonic, benzenesulfonic and p-toluenesulfonic acids and inorganic acids such as hydrochloric, sulfuric, phosphoric and sulfamic acids. Physiologically acceptable salts of a compound with a hydroxy group include the anion of said compound in combination with a suitable cation such as $Na^+$ $NH_4^+$, and $NW_4^+$ (wherein W is a $C_{1-4}$ alkyl group).

The above compounds according to the invention may be employed in combination with other therapeutic agents for the treatment or prophylaxis of the above infections or conditions. Examples of such further therapeutic agents include agents that are effective for the treatment or prophylaxis of viral infections or associated conditions such as 3'-azido-3'-deoxy-thymidine (zidovudine), 2',3'-dideoxynucleosides such as 2',3'-dideoxy-cytidine, 2',3'-dideoxy adenosine and 2',3'-dideoxyinosine, acyclic nucleosides (eg acyclovir), interferons such as α-interferon, renal excretion inhibitors such as probenicid, nucleoside transport inhibitors such as dipyridamole, dialzep, mio-, lido- or foluflazine or hexobendine as well as immunomodulators such as interleukin II and granulocyte macrophage colony stimulating factors, soluble $CD_4$ or genetically engineered derivatives thereof, and phosphonoformic acid. The component compounds of such combination therapy may be administered simultaneously, in either separate or combined formulations, or at different times, e.g. sequentially such that a combined effect is achieved.

The compounds according to the invention, also referred to herein as the active ingredient. may be administered for therapy by any suitable route including oral, rectal, nasal, topical (including buccal and sublingual), vaginal and parenteral (including subcutaneous, intramuscular, intravenous and intradermal). It will be appreciated that the preferred route will vary with the condition and age of the recipient, the nature of the infection and the chosen active ingredient.

In general a suitable dose will be in the range of 3.0 to 120 mg per kilogram body weight of the recipient per day, preferably in the range of 6 to 90 mg per kilogram body weight per day and most preferably in the range 15 to 60 mg per kilogram body weight per day. The desired dose is preferably presented as two, three, four, five, six or more sub-doses administered at appropriate intervals throughout the day. These sub-doses may be administered in unit dosage forms, for example, containing 10 to 1500 mg, preferably 20 to 1000 mg, and most preferably 50 to 700 mg of active ingredient per unit dosage form.

Ideally, the active ingredient should be administered to achieve peak plasma concentrations of the active compound of from about 1 to about 75μM, preferably about 2 to 50μM, most preferably about 3 to about 30μM. This may be achieved, for example, by the intravenous injection of a 0.1 to 5% solution of the active ingredient, optionally in saline, or orally administered as a bolus containing about 1 to about 100 mg/kg of the active ingredient. Desirable blood levels may be maintained by a continuous infusion to provide about 0.01 to about 5.0 mg/kg/hour or by intermittent infusions containing about 0.4 to about 15 mg/kg of the active ingredient.

While it is possible for the active ingredient to be administered alone it is preferable to present it as a pharmaceutical formulation. The formulations of the present invention comprise at least one active ingredient, as defined above, together with one or more acceptable carriers thereof and optionally other therapeutic agents. Each carrier must be "acceptable" in the sense of being compatible with the other ingredients of the formulation and not injurious to the patient. Formulations include those suitable for oral, rectal, nasal, topical (including buccal and sublingual), vaginal or parenteral (including subcutaneous, intramuscular, intravenous and intradermal) administration. The formulations may conveniently be presented in unit dosage form and may be prepared by any methods well known in the art of pharmacy. Such methods include the step of bringing into association the active ingredient with the carrier which constitutes one or more accessory ingredients. In general, the formulations are prepared by uniformly and intimately bringing into association the active ingredient with liquid carriers or finely divided solid carriers or both, and then if necessary shaping the product.

Formulations of the present invention suitable for oral administration may be presented as discrete units such as capsules, cachets or tablets each containing a predetermined amount of the active ingredient; as a powder or granules; as a solution or a suspension in an aqueous or non-aqueous liquid; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder (e.g. povidone, gelatin, hydroxypropylmethyl cellulose), lubricant, inert diluent, preservative, disintegrant (e.g. sodium starch glycollate, cross-linked povidone, cross-linked sodium carboxymethyl cellulose) surface-active or dispersing agent. Moulded tablets may be made by moulding in a suitable machine a mixture of the powdered compound moistened with an inert liquid diluent. The tablets may optionally

be coated or scored and may be formulated so as to provide slow or controlled release of the active ingredient therein using, for example, hydroxypropylmethyl cellulose in varying proportions to provide the desired release profile. Tablets may optionally be provided with an enteric coating, to provide release in parts of the gut other than the stomach. This is particularly advantageous for purine nucleoside derivatives as such compounds are susceptible to acid hydrolysis.

Formulations suitable for topical administration in the mouth include lozenges comprising the active ingredient in a flavoured basis, usually sucrose and acacia or tragacanth; pastilles comprising the active ingredient in an inert basis such as gelatin and glycerin, or sucrose and acacia; and mouthwashes comprising the active ingredient in a suitable liquid carrier.

Formulations for rectal administration may be presented as a suppository with a suitable base comprising for example cocoa butter or a salicylate.

Formulations suitable for vaginal administration may be presented as pessaries, tampons, creams, gels, pastes, foams or spray formulations containing in addition to the active ingredient such carriers as are known in the art to be appropriate.

Formulations suitable for parenteral administration include aqueous and non- aqueous isotonic sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose sealed containers, for example, ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets of the kind previously described.

Preferred unit dosage formulations are those containing a daily dose or unit, daily sub-dose, as herein above recited, or an appropriate fraction thereof. of an active ingredient.

The compounds according to the invention may also be presented for use in the form of veterinary formulations, which may be prepared, for example, by methods that are conventional in the art.

It should be understood that in addition to the ingredients particularly mentioned above the formulations of this invention may include other agents conventional in the art having regard to the type of formulation in question, for example, those suitable for oral administration may include such further agents as sweeteners, thickeners and flavouring agents.

The present invention further includes a process for the preparation of a compound according to the invention which comprises either:

A) treating a compound of formula

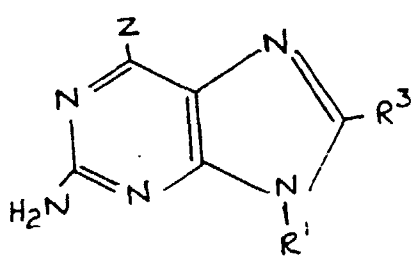

(II)

wherein $R^1$ is as hereinbefore defined and Z represents a precursor group for the said $R^2$ group with an agent or under conditions serving to convert the precursor Z group to the desired $R^2$ group); or Z represents a thio group onto which may be substituted an appropriate group to form a compound of formula (I) wherein $R^2$ is (2-cyclohexen-1-yl)thio or;

B) reacting a compound of formula

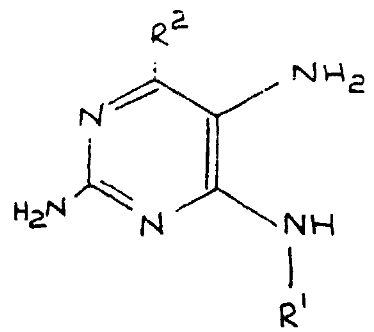

(III)

(wherein $R^1$ and $R^2$ are hereinbefore defined) with an agent serving to effect formation of the imidazole ring in the desired compound of formula (I); or where a pharmaceutically acceptable derivative of a compound of formula (I) is formed, converting the said derivative to a compound of formula (I).

Process A) above may be carried out in conventional manner, for example, by treatment of a compound of formula (II) in which Z represents a leaving group, e.g. a halo such as a chloro group, with, for example. an alkali metal (e.g. sodium) or alkali metal hydride (e.g. sodium hydride) and an appropriate alcohol at reflux or a temperature greater than 50°C preferably in an organic solvent.

Alternatively the compound of formula (II) may be treated with an appropriate amine or amino hydrochloride to introduce a substituted amino group as defined above at reflux or at a temperature greater than 50°C preferably in the presence of an organic solvent, for example methanol, ethanol. Alternatively, a compound of formula (II) wherein Z is a thio group may be treated with an appropriate halide or halo under nitrogen.

Process B) may be carried out, e.g. by reacting a compound of formula (III) with formic acid or a reactive formic acid derivative, triethyl orthoformate or diethoxymethyl acetate, in a solvent such as a dimethylacetamide or acetonitrile at an elevated temperature, preferably at 75-90°C. This reaction is conveniently effected by the addition of slightly more than one equivalent of a strong anhydrous acid, with 1.1 equivalents of ethanesulfonic acid per equivalent of compound of formula (III), in which case lower temperatures, 25°C., are used.

In process A) the starting material of formula (II) may be prepared, for example, by firstly cyclising a compound of formula (III) above in an analogous manner to that described for process B) above. Alternatively, (±)-9-(cis-4-(hydroxymethyl)-2-cyclopentenyl)guanine prepared e.g. as described in AU-A-28671/89 incorporated herein by reference from aristeromycin, may be converted to compounds of formula (I).

Other reagents may be useful for cyclisation of compounds of formula (III) to give compounds of formula (I) where $R^3$ is not hydrogen. For example triethyl or trimethylorthoacetate with acetic anhydride at 70-120°C for several hours gives $R^3$=$CH_3$ (see H.C. Koppel and R.K. Robins, J.Org.Chem. 1958, 1457), $R^3$=$NH_2$ may be obtained by cylisation with ethoxycarbonyl isothiocyanate (see R. Esmail and F.Kurzer, Synthesis 1975, 301; L.B.Towsend, et al., J. Heterocyclic Chem. 1984, 21, 1245). This initially gives $R^3$=$NHC_2$ Et which is then hydrolysed in base (e.g. aqueous sodium hydroxide) to $R^3$=$NH_2$. Cyclisation with potassium ethylanthate (W.T. Stolle, J.C. Sih, R.S.P. Hsi, J. Lable. Compound Radiopharm. 1988, 891) in ethanol at 80°C gives $R^3$=SH. Alkylation of the SH with alkyl halides and base (e.g. potassium carbonate in DMF) gives $R^3$=SMe. SEt.

A compound of formula (I) may be converted into a pharmaceutically acceptable ester by reaction wich an appropriate esterifying agent, e.g. an acid halide or anhydride. The compound of formula (I), including esters thereof, may be converted into pharmaceutically acceptable salts thereof in conventional manner, e.g. by treatment with an appropriate acid. An ester or salt of a compound of formula (I) may be converted into the parent compound, e.g. by hydrolysis.

The enantiomers of the compounds of formula (I) may be resolved or isolated in conventional manner, e.g. by chromatographic separation or diastereomeric esters prepared by acylation of the hydroxyl on the cyclopentenyl moiety with appropriate optically active carboxylic acid derivatives as, e.g., with naproxen (J.Org.Chem., 51, 1287 (1986)). The cyclopentenyl precursors of the compounds of formula (III), may also be resolved by fractional crystallization of salts formed with optically active carboxylic acids (e.g. di-benzoyl-D-tartaric acid and its derivatives). Alternatively, enzymatic resolution may be achieved as in J.Med.Chem., 30, 746 (1987) and J.Med.Chem. 28, 1385 (1985).

The following Examples are intended for illustration only and are not intended to limit the scope of the invention in any way. The term 'active ingredient' as used in the Examples means a compound of formula (I) or a pharmaceutically acceptable derivative thereof.

Example 1

(±)-cis-4-(2-Amino-6-(2-cyclohexen-1-yl)thio)-9H-purin-9-yl)-2-cyclopenten-1-methanol

To a solution of (±)-cis-2-amino-1,9-dihydro-9-[(4-hydroxymethyl)-2-cyclopenten-1-yl]-6H-purin-6-thione (0.50g, 1.89mMol) from Example 5 in 1N NaOH (1.89ml) was added 3-bromocyclohexene (0.305g, 1.89mMol) and dioxane (2mL). The solution was stirred under nitrogen for 2 hours and extracted with $CHCl_3$ (3x50mL). $CHCl_3$ extracts were dried ($MgSO_4$) and concentrated to yellow oil. Chromatography on silica gel gave title compound, which was eluted with 10% Me0H-$CHCl_3$ and solidified to a yellow powder in $CH_3CN$, Mp.138-140°C;
'H-NMR (DMSO-$d_6$) δ; 7.82 (S, 1, H-8) 6.46 (br S, 2, $NH_2$), 6.15 and 5.85 (both m, overlapping 5.90-5.70, m, total 4, 2',3' and cyclohexene CH-CH), 5.40 (m, 1, (H-N), 4.90 (br m, 1, S-CH), 4.70 (t,J-5.3Hz, 1, OH), 3.45 (m, 2, $CH_2$-0), 2.85 (br m, 1, CH), 2.70-2.55 (m, 1, 0.5 $CH_2$), 2.10-1.50 (m, 7, 3 $CH_2$ plus 0.5 $CH_2$)
Anal. calc. for $C_{17}H_{21}N_50S$: C,59.45; H,6.16; N,20.39; S,9.34
Found: C,59.17; H,6.18; N,20.31; S,9.25

Example 2

(%)-cis-4-(2-Amino-6-(2-cyclopenten-1-yl amino)-9H-purin-9-yl)-2-cyclopentene-1-methanol

A solution of (±)-cis-4-(2-Amino-6-chloro-9H-purin-9-yl)-2-cyclopentene-1-methanol hydrate (0.53g, 1.87mMol) from Example 4, triethylamine (2.00g, 20mMol) and 3-amino cyclopentene hydrochloride (463mg, 3.87mMol) in 10mL of ethanol was stirred at reflux for 17 hours. The solution was allowed to cool to room temperature before the addition of 2mL of 1.0N NaOH. Concentration of the solution afforded the crude product which was purified by elution from a silica gel column using 5% methanol in chloroform (0.30g, 48.0%). Crystallization of such a sample from ethanol-acetonitrile gave an off-white powder, Mp. 143-146°C;
'H-NMR ($Me_2S0$-$d_6$) δ 7.58 (s, 1H, purine H8), 6.97 (br d J=12Hz, 1H, NH), 6.10 (m, 1H, =CH), 5.89-5.72 (m overlapping br s at 5.86, 5H, 3=CH,$NH_2$), 5.37 (m overlapping br m at 5.2; 4.73 (unresolved t, 1H, OH), 3.42 (m, 2H, $OCH_2$), 2.84 (br m, 1H, CH) 2.66-2.14 (m, overlapping DMS0, 3 x 0.5 $CH_2$), 1.80-1.48 (m, 2H, 2 x 0.5$CH_2$).
Anal. calc. for $C_{16}H_{20}N_6O$: C,61.52; H,6.45; N,26.9
Found: C,61.62; H,6.47; N,26.88.

Example 3

(%)-cis-4-[2-Amino-6-((8-Aminooctyl)amino)-9H-purin-9-yl)-2-cyclopentene-1-methanol

A solution of (±)-(cis)-4-(2-amino-6-chloro-9H-purin-9-yl)-2-cyclopentene-1-methanol (272 mg, 1.00 mmol) from Example 4 and 1,8-diaminooctane (1.44 g, 10 mmol) in absolute ethanol (15 mL) was refluxed under nitrogen for 1.5 hours. The solution was concentrated, cooled, and the resulting precipitate filtered and crystallised from i-propanol to give title compound as white solid (160 mg, 42%), m.p. 125-127°C.
'H-NMR (DMS0-$d_6$) δ 7.57 (s, 1, purine H-8), 7.10 (br s, 1, NH), 6.10 and 5.85 (both m, 2, CH=CH), 5.74 (br s, 1, $NH_2$), 5.40 (m, 1, CH-N), 4.75 (br m, 1, OH), 3.7-3.0 (br m overlapped by $H_20$, $CH_2$-0, 2N$CH_2$, $NH_2$), 2.85 (br m, 1, H-4'), 2.7-2.4 (m overlapping $d_5$-DMSO, 0.5 $CH_2$ and $CH_2$ $CH_2$N), 1.55 (m, 3.6, $CH_2$ $CH_2$N and 0.5 $CH_2$), 1.25 (br m, 8, 4$CH_2$).
Anal. calc. for $C_{19}H_{31}N_70.0.3H_20$: C,60.23; H,8.41; N,25.88.
Found: C,60.42; H,8.26; N,25.78.

Example 4

(%)-cis-4-(2-Amino-6-((cyclopropymethyl)amino)-9H-purin-9-yl)-2-cyclopentene-1-methanol

(±)-(cis)-(2-Amino-6-chloro-9H-purin-9-yl)-2-cyclopentene-1-methanol from Example 4 (0.544 g, 2 mmol), aminomethylcyclopropane hydrochloride (0.323 g, 3 mmol), triethylamine (0.607 g, 6 mmol) and methanol (15 mL) were heated in a Parr bomb for 12 hours. During this time, an additional aminomethylcyclopropane hydrochloride (0.108 g, 1 mmol) and triethylamine (0.101 g, 1 mmol), were added. 1 N NaOH (2 ml) was added and solution concentrated under vacuum, dried by evaporation of ethanol, and chromatographed on silica gel. Title compound was eluted in 5% methanol-chloroform; white powder after crystallisation from acetonitrile (0.425 g, 71%), m.p. 182-183°C.
'H-NMR (DMSO-$d_6$) δ 7.58 (s, 1, H-8), 6.10 and 5.85 (2 m, overlapping s at 5.77, total 4, CH=CH and $NH_2$), 5.40 (br m, 1, CH-N), 4.73 (t, J=5.3, 1, OH), 3.45 (m, 2, $CH_2$-0), 3.40-3.20 (m, overlapping $H_2O$, $CH_2$-NH), 2.85 (br m, 1, CH), 2.70-2.50 (m, overlapping solvent, 0.5 $CH_2$), 1.70-1.50 (m, 1, 0.5 $CH_2$, 1.20-1.0 (m, l, CH-$CH_2$NH), 0.45-0.20 (m, 2,

$CH_2$-$CH_2$ cyclopropyl).
Anal. calc. for $C_{15}H_{20}N_60$: C,59.98; H,6.71; N,27.98.
Found: C,59.90, 59,83; H,6.72, 6.76; N,27.91.

Example A

Tablet Formulations

The following formulations A, B and C are prepared by wet granulation of the ingredients with a solution of povidone, followed by addition of magnesium stearate and compression.

| Formulation A | | | |
|---|---|---|---|
| | | mg/tablet | mg/tablet |
| (a) | Active ingredient | 250 | 250 |
| (b) | Lactose B.P. | 210 | 26 |
| (c) | Povidone B.P. | 15 | 9 |
| (d) | Sodium Starch Glycollate | 20 | 12 |
| (e) | Magnesium Stearate | 5 | 3 |
| | | 500 | 300 |

| Formulation B | | | |
|---|---|---|---|
| | | mg/tablet | mg/tablet |
| (a) | Active ingredient | 250 | 250 |
| (b) | Lactose | 150 | - |
| (c) | Avicel PH 101 | 60 | 26 |
| (d) | Povidone B.P. | 5 | 9 |
| (e) | Sodium Starch Glycollate | 20 | 12 |
| (f) | Magnesium Stearate | 5 | 3 |
| | | 500 | 300 |

| Formulation C | |
|---|---|
| | mg/tablet |
| Active ingredient | 100 |
| Lactose | 200 |
| Starch | 50 |
| Povidone | 5 |
| Magnesium stearate | 4 |
| | 359 |

The following formulations, D and E, are prepared by direct compression of the admixed ingredients. The lactose in formulation E is of the direct compression type (Dairy Crest - "Zeparox").

| Formulation D | |
|---|---|
| | mg/tablet |
| Active ingredient | 250 |
| Pregelatinised Starch NF15 | 150 |
| | 400 |

| Formulation E | |
|---|---|
| | mg/tablet |
| Active ingredient | 250 |
| Lactose | 150 |
| Avicel | 100 |
| | 500 |

Formulation F (Controlled Release Formulation)

The formulation is prepared by wet granulation of the ingredients (below) with a solution of povidone followed by the addition of magnesium stearate and compression.

| | | mg/tablet |
|---|---|---|
| (a) | Active ingredient | 500 |
| (b) | Hydroxypropylmethylcellulose (Methocel K4M Premium) | 112 |
| (c) | Lactose B.P. | 53 |
| (d) | Povidone B.P. | 28 |
| (e) | Magnesium Stearate | 7 |
| | | 700 |

Drug release takes place over a period of about 6-8 hours and is complete after 12 hours.

Example B

Capsule Formulations

Formulation A

A capsule formulation is prepared by admixing the ingredients of Formulation D in Example A above and filling into a two-part hard gelatin capsule. Formulation B (infra) is prepared in a similar manner.

| Formulation B | | |
|---|---|---|
| | | mg/capsule |
| (a) | Active ingredient | 250 |
| (b) | Lactose B.P. | 143 |
| (c) | Sodium Starch Glycollate | 25 |
| (d) | Magnesium Stearate | 2 |
| | | 420 |

| Formulation C | | |
|---|---|---|
| | | mg/capsule |
| (a) | Active ingredient | 250 |
| (b) | Macrogol 4000 B.P. | 350 |
| | | 600 |

Capsules of formulation C are prepared by melting the Macrogol 4000 BP, dispersing the active ingredient in the melt and filling the melt into a two-part hard gelatin capsule.

| Formulation D | |
|---|---|
| | mg/capsule |
| Active ingredient | 250 |
| Lecithin | 100 |
| Arachis Oil | 100 |
| | 450 |

Capsules of formulation D are prepared by dispersing the active ingredient in the lecithin and arachis oil and filling the dispersion into soft, elastic gelatin capsules.

Formulation E (Controlled Release Capsule)

The following controlled release capsule formulation is prepared by extruding ingredients a, b and c using an extruder, followed by spheronisation of the extrudate and drying. The dried pellets are then coated with release- controlling membrane (d) and filled into a two-piece, hard gelatin capsule.

| | | mg/capsule |
|---|---|---|
| (a) | Active ingredient | 250 |
| (b) | Microcrystalline Cellulose | 125 |
| (c) | Lactose B.P. | 125 |
| (d) | Ethyl Cellulose | 13 |
| | | 513 |

Example C

Injectable Formulation

| Formulation A | |
|---|---|
| Active ingredient | 0.200g |
| Hydrochloric acid solution, 0.1M, or Sodium hydroxide solution, 0.1M q.s. to pH | 4.0 to 7.0 |
| Sterile water q.s. to | 10ml |

The active ingredient is dissolved in most of the water (35°-40°C) and the pH adjusted to between 4.0 and 7.0 with the hydrochloric acid or the sodium hydroxide as appropriate. The batch is then made up to volume with the water and filtered through a sterile micropore filter into a sterile 10ml amber glass vial (type 1) and sealed with sterile closures and overseals.

| Formulation B. | |
|---|---|
| Active ingredient | 0.125 g |
| Sterile, pyrogen-free, pH 7 phosphate buffer, q.s. to | 25 ml |

Example D

| Intramuscular injection | |
|---|---|
| Active ingredient | 0.20 g |
| Benzyl Alcohol | 0.10 g |
| Glycofurol 75 | 1.45 g |

(continued)

| Intramuscular injection | |
|---|---|
| Water for Injection q.s. to | 3.00 ml |

The active ingredient is dissolved in the glycofurol. The benzyl alcohol is then added and dissolved, and water added to 3 ml. The mixture is then filtered through a sterile micropore filter and sealed in sterile 3 ml amber glass vials (type 1).

Example E

| Syrup | |
|---|---|
| Active ingredient | 0.25 g |
| Sorbitol Solution | 1.50 g |
| Glycerol | 2.00 g |
| Sodium Benzoate | 0.005 g |
| Flavour, Peach 17.42.3169 | 0.0125 ml |
| Purified Water q.s. to | 5.00 ml |

The active ingredient is dissolved in a mixture of the glycerol and most of the purified water. An aqueous solution of the sodium benzoate is then added to the solution, followed by addition of the sorbitol solution and finally the flavour. The volume is made up with purified water and mixed well.

Example F

| Suppository | |
|---|---|
| | mg/suppository |
| Active ingredient (63µm)* | 250 |
| Hard Fat, BP (Witepsol H15 - Dynamit NoBel) | 1770 |
| | 2020 |

*The active ingredient is used as a powder wherein at least 90% of the particles are of 63µm diameter or less.

One-fifth of the Witepsol H15 is melted in a steam-jacketed pan at 45°C maximum. The active ingredient is sifted through a 200 m sieve and added to the molten base with mixing, using a silverson fitted with a cutting head, until a smooth dispersion is achieved. Maintaining the mixture at 45°C, the remaining Witepsol H15 is added to the suspension and stirred to ensure a homogenous mix. The entire suspension is passed through a 250 m stainless steel screen and, with continuous stirring, is allowed to cool to 40°C, At a temperature of 38°C to 40°C, 2.02g of the mixture is filled into suitable, 2 ml plastic moulds. The suppositories are allowed to cool to room temperature.

Example G

| Pessaries | |
|---|---|
| | mg/pessary |
| Active ingredient (63µm) | 250 |
| Anhydrate Dextrose | 380 |
| Potato Starch | 363 |

(continued)

| Pessaries | |
|---|---|
| | mg/pessary |
| Magnesium Stearate | 7 |
| | 1000 |

The above ingredients are mixed directly and pessaries prepared by direct compression of the resulting mixture.

Antiviral Activity

The compounds are tested for anti-HIV activity in $MT_4$ cells according to the method described by Averett, D. R., Journal of Virological Methods, 23, 1989, pp263-276. Activity is expressed as $IC_{50}$ values in µM.

Determination of anti-HBV activity is carried out by testing the ability of a compound to prevent replication of duck HBV in vitro, in the manner described by Tuttleman, Pugh and Summers (J. Virology, 58: 17-25, 1986). Duck hepatocytes are placed in culture, and infected with duck HBV. Three days after infection, the infected cells are exposed to various concentrations of the test compound for an additional period of eight days. After this exposure, DNA is extracted from each culture of infected cells and compound, and the amount of viral DNA is specifically determined and compared with that obtained from similar cultures lacking the test compound.

Toxicity Data

Determination of Growth Inhibition of Uninfected Mammalian Cells

The capability of candidate compounds to inhibit the growth of D98 cells (human) and L cells (murine) is measured by determination of cell number following three days exposure of a standard number of cells to various dilutions of compound (Rideout, J.L., Krenitsky, T.A., Koszalka, G.W., Cohn, N.K., Chao, E.Y. Elion, G.B., Latter, V.S., and Williams, R.B. (1982) J. Med. Chem. 25: 1040-1044). The cell number is then compared to the number obtained in the absence of compound. Cell enumeration is performed by either direct particle counts following trypsinization of the monolayer, or by spectrophotometric determination of the amount of vital stain taken up by the cells. Comparable results are obtained with both methods.

Data Analysis

The concentration of compound resulting in 50% of control values ($IC_{50}$) is calculated either by direct interpolation from graphs of the log of the compound concentration versus the percent of control value, or from a computer program which analyses the data according to the same algorithm. Data in the range of 20% to 80% of control are used in these calculations. Compounds were tested in D98 cells and found to have an $IC_{50}$ value greater than 100µm.

**Claims**

**Claims for the following Contracting States : AT, BE, CH, DE, DK, FR, GB, IT, LI, LU, NL, SE**

1. A compound selected from:
   (±)-cis-4-(2-amino-6-(2-cyclohexen-1-yl)thio)-9H-purin-9-yl)-2-cyclopenten-1-methanol;
   (±)-cis-4-(2-amino-6-(2-cyclohexen-1-yl amino)-9H-purin-9-yl)-2-cyclopenten-1-methanol;
   (±)-cis-4-(2-amino-6-((8-aminooctyl)amino)-9H-purin-9-yl)-2-cyclopenten-1-methanol; and
   (±)-cis-4-(2-amino-6-((cyclopropylmethyl)amino)-9H-purin-9-yl)-2-cyclopenten-1-methanol.

2. A compound as claimed in claim 1 for use in medical therapy.

3. Use of a compound as defined in claim 1 or a salt, ester or salt of such an ester thereof in the manufacture of a medicament for the treatment or prophylaxis of a viral infection.

4. Use of a compound as defined in claim 1 or a salt, ester or salt of such an ester thereof in the manufacture of a medicament for the treatment or prophylaxis of a retroviral or Hepatitis B viral infection.

5. Use of a compound as defined in claim 1 or a salt, ester or salt of such an ester thereof in the manufacture of a medicament for the treatment or propylaxis of an HIV infection.

6. A pharmaceutical formulation comprising a compound as defined in claim 1 or a pharmaceutically acceptable salt thereof, and a pharmaceutically acceptable carrier.

7. A process for the preparation of compounds of formula (I):

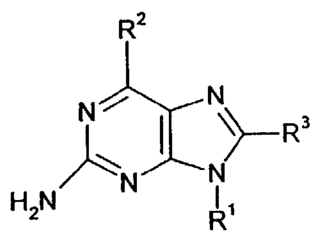

(I)

wherein $R^1$ represents

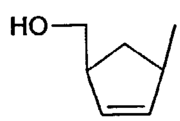

(A)

$R^2$ represents (2-cyclohexen-1-yl)thio, (2-cyclopenten-1-yl)amino, (8-aminooctyl)amino or (cyclopropylmethyl) amino; and $R^3$ is hydrogen which comprises either

A) treating a compound of formula (II)

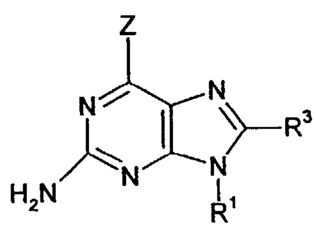

(II)

wherein $R^1$ and $R^3$ are as herein before defined and Z represents a precursor group for the said $R^2$ group with an agent or under conditions serving to convert the precursor Z group to the desired $R^2$ group; or Z represents a thio group onto which may be substituted an appropriate group to form a compound of formula (I) wherein $R^2$ is (2-cyclohexen-1-yl)thio or;

B) reacting a compound of formula

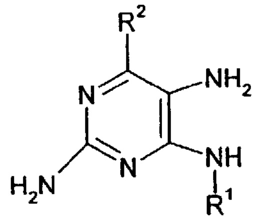

(wherein $R^1$ and $R^2$ are hereinbefore defined) with an agent serving to effect formation of the imidazole ring in the desired compound of formula (I): or where a pharmaceutically acceptable derivative of a compound of formula (I) is formed, converting the said derivative to a compound of formula (I).

**Claims for the following Contracting State : ES**

1. A process for the preparation of compounds of formula (I):

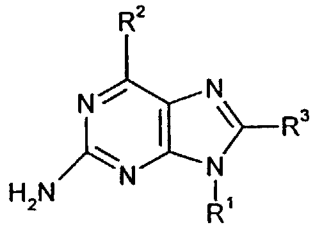

(I)

wherein $R^1$ represents

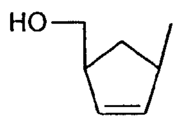

(A)

$R^2$ represents (2-cyclohexen-1-yl)thio, (2-cyclopenten-1-yl)amino, (8-aminooctyl)amino or (cyclopropylmethyl) amino; and $R^3$ is hydrogen which comprises either

A) treating a compound of formula (II)

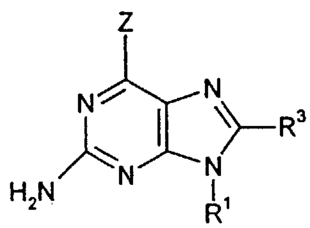

(II)

wherein $R^1$ and $R^3$ are as herein before defined and Z represents a precursor group for the said $R^2$ group with an agent or under conditions serving to convert the precursor Z group to the desired $R^2$ group; or Z represents a thio group onto which may be substituted an appropriate group to form a compound of formula (I) wherein $R^2$ is (2-cyclohexen-1-yl)thio or;

B) reacting a compound of formula

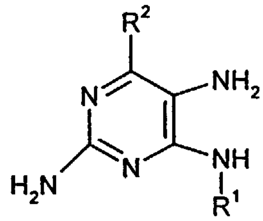

(wherein $R^1$ and $R^2$ are hereinbefore defined) with an agent serving to effect formation of the imidazole ring in the desired compound of formula (I): or where a pharmaceutically acceptable derivative of a compound of formula (I) is formed, converting the said derivative to a compound of formula (I).

**2.** A compound selected from:
(±)-cis-4-(2-amino-6-(2-cyclohexen-1-yl)thio)-9H-purin-9-yl)-2-cyclopenten-1-methanol;
(±)-cis-4-(2-amino-6-(2-cyclohexen-1-yl amino)-9H-purin-9-yl)-2-cyclopenten-1-methanol;
(±)-cis-4-(2-amino-6-((8-aminooctyl)amino)-9H-purin-9-yl)-2-cyclopenten-1-methanol; and
(±)-cis-4-(2-amino-6-((cyclopropylmethyl)amino)-9H-purin-9-yl)-2-cyclopenten-1-methanol.

**3.** A compound as claimed in claim 2 for use in medical therapy.

**4.** Use of a compound as defined in claim 2 or a salt, ester or salt of such an ester thereof in the manufacture of a medicament for the treatment or prophylaxis of a viral infection.

**5.** Use of a compound as defined in claim 2 or a salt, ester or salt of such an ester thereof in the manufacture of a medicament for the treatment or prophylaxis of a retroviral or Hepatitis B viral infection.

**6.** Use of a compound as defined in claim 2 or a salt, ester or salt of such an ester thereof in the manufacture of a medicament for the treatment or propylaxis of an HIV infection.

**7.** A pharmaceutical formulation comprising a compound as defined in claim 2 or a pharmaceutically acceptable salt thereof, and a pharmaceutically acceptable carrier.

## Patentansprüche

### Patentansprüche für folgende Vertragsstaaten : AT, BE, CH, DE, DK, FR, GB, IT, LI, LU, NL, SE

**1.** Verbindung, ausgewählt aus:
(±)-cis-4-(2-Amino-6-((2-cyclohexen-1-yl)thio)-9H-purin-9-yl)-2-cyclopenten-1-methanol,
(±)-cis-4-(2-Amino-6-((2-cyclohexen-1-yl)amino)-9H-purin-9-yl)-2-cyclopenten-1-methanol,
(±)-cis-4-(2-Amino-6-((8-aminooctyl)amino)-9H-purin-9-yl)-2-cyclopenten-1-methanol und
(±)-cis-4-(2-Amino-6-((cyclopropylmethyl)amino)-9H-purin-9-yl)-2-cyclopenten-1-methanol.

**2.** Verbindung gemäss Anspruch 1 zur Verwendung in der medizinischen Therapie.

**3.** Verwendung einer Verbindung wie in Anspruch 1 definiert oder eines Salzes, Esters oder Salzes eines solchen Esters davon bei der Herstellung eines Medikaments zur Behandlung oder Prophylaxe einer Virusinfektion.

**4.** Verwendung einer Verbindung wie in Anspruch 1 definiert oder eines Salzes, Esters oder Salzes eines solchen Esters davon bei der Herstellung eines Medikaments zur Behandlung oder Prophylaxe einer Retrovirus- oder Hepatitis B-Virusinfektion.

**5.** Verwendung einer Verbindung wie in Anspruch 1 definiert oder eines Salzes, Esters oder Salzes eines solchen Esters davon bei der Herstellung eines Medikaments zur Behandlung oder Prophylaxe einer HIV-Infektion.

6. Pharmazeutische Formulierung, umfassend eine Verbindung wie in Anspruch 1 definiert oder ein pharmazeutisch akzeptables Salz davon und einen pharmazeutisch akzeptablen Träger.

7. Verfahren zur Herstellung von Verbindungen der Formel (I) :

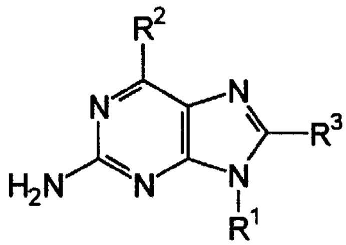

(I)

worin $R^1$

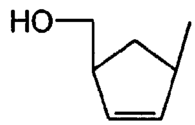

(A)

darstellt;
$R^2$ (2-Cyclohexen-1-yl)thio, (2-Cyclopenten-1-yl)amino, (8-Aminooctyl)amino oder (Cyclopropylmethyl)amino darstellt; und $R^3$ Wasserstoff ist;
welches entweder umfasst:

(A) Behandeln einer Verbindung der Formel (II):

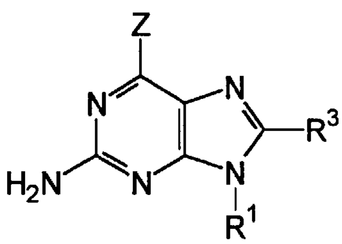

(II)

worin $R^1$ und $R^3$ wie zuvor definiert sind und Z eine Vorstufengruppe für die $R^2$-Gruppe darstellt, mit einem Agens oder unter Bedingungen, die dazu dienen, die Vorstufengruppe Z zur gewünschten $R^2$-Gruppe zu konvertieren; oder worin Z eine Thiogruppe darstellt, an die eine entsprechende Gruppe zur Bildung einer Verbindung der Formel (I) substituiert werden kann, worin $R^2$ (2-Cyclohexen-1-yl)thio ist; oder

(B) Umsetzen einer Verbindung der Formel:

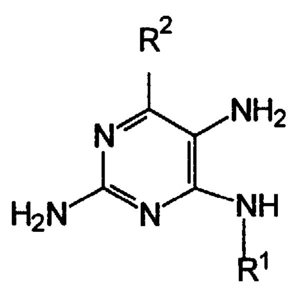

(worin $R^1$ und $R^2$ wie zuvor definiert sind) mit einem Agens, das dazu dient, die Bildung des Imidazolringes in der gewünschten Verbindung der Formel (I) zu bewirken; oder wenn ein pharmazeutisch akzeptables Derivat einer Verbindung der Formel (I) gebildet wird, Konvertieren des Derivats zu einer Verbindung der Formel (I).

**Patentansprüche für folgenden Vertragsstaat : ES**

1. Verfahren zur Herstellung von Verbindungen der Formel (I):

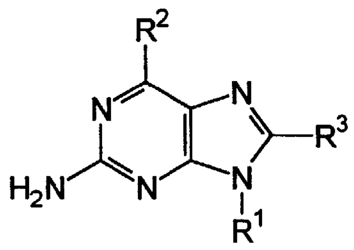

(I)

worin $R^1$

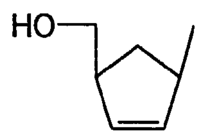

(A)

darstellt;
$R^2$ (2-Cyclohexen-1-yl)thio, (2-Cyclopenten-1-yl)amino, (8-Aminooctyl)amino oder (Cyclopropylmethyl)amino darstellt; und $R^3$ Wasserstoff ist;
welches entweder umfasst:

(A) Behandeln einer Verbindung der Formel (II):

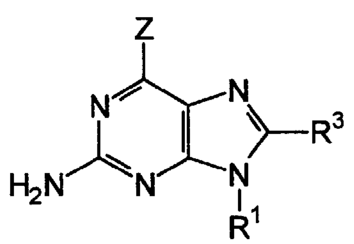

(II)

worin $R^1$ und $R^3$ wie zuvor definiert sind und Z eine Vorstufengruppe für die $R^2$-Gruppe darstellt, mit einem Agens oder unter Bedingungen, die dazu dienen, die Vorstufengruppe Z zur gewünschten $R^2$-Gruppe zu konvertieren; oder worin Z eine Thiogruppe darstellt, an die eine entsprechende Gruppe zur Bildung einer Verbindung der Formel (I) substituiert werden kann, worin $R^2$ (2-Cyclohexen-1-yl)thio ist; oder

(B) Umsetzen einer Verbindung der Formel:

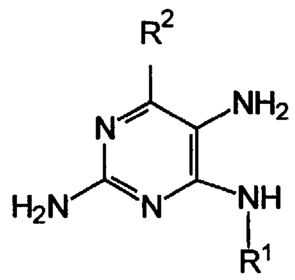

(worin $R^1$ und $R^2$ wie zuvor definiert sind) mit einem Agens, das dazu dient, die Bildung des Imidazolringes in der gewünschten Verbindung der Formel (I) zu bewirken; oder wenn ein pharmazeutisch akzeptables Derivat einer Verbindung der Formel (I) gebildet wird, Konvertieren des Derivats zu einer Verbindung der Formel (I).

**2.** Verbindung, ausgewählt aus:
(±)-cis-4-(2-Amino-6-((2-cyclohexen-1-yl)thio)-9H-purin-9-yl)-2-cyclopenten-1-methanol,
(±) -cis-4-(2-Amino-6-((2-cyclohexen-1-yl)amino)-9H-purin-9-yl)-2-cyclopenten-1-methanol,
(±)-cis-4-(2-Amino-6-((8-aminooctyl)amino)-9H-purin-9-yl)-2-cyclopenten-1-methanol und
(±)-cis-4-(2-Amino-6-((cyclopropylmethyl)amino)-9H-purin-9-yl)-2-cyclopenten-1-methanol.

**3.** Verbindung gemäss Anspruch 2 zur Verwendung in der medizinischen Therapie.

**4.** Verwendung einer Verbindung wie in Anspruch 2 definiert oder eines Salzes, Esters oder Salzes eines solchen Esters davon bei der Herstellung eines Medikaments zur Behandlung oder Prophylaxe einer Virusinfektion.

**5.** Verwendung einer Verbindung wie in Anspruch 2 definiert oder eines Salzes, Esters oder Salzes eines solchen Esters davon bei der Herstellung eines Medikaments zur Behandlung oder Prophylaxe einer Retrovirus- oder Hepatitis B-Virusinfektion.

**6.** Verwendung einer Verbindung wie in Anspruch 2 definiert oder eines Salzes, Esters oder Salzes eines solchen Esters davon bei der Herstellung eines Medikaments zur Behandlung oder Prophylaxe einer HIV-Infektion.

**7.** Pharmazeutische Formulierung, umfassend eine Verbindung wie in Anspruch 2 definiert oder ein pharmazeutisch akzeptables Salz davon und einen pharmazeutisch akzeptablen Träger.

## Revendications

### Revendications pour les Etats contractants suivants : AT, BE, CH, DE, DK, FR, GB, IT, LI, LU, NL, SE

**1.** Composé choisi parmi :
le (±)cis-4-(2-amino-6-(2-cyclohexén-1-yl)thio)-9H-purin-9-yl)-2-cyclopentène-1-méthanol;
le (±)cis-4-(2-amino-6-(2-cyclohexén-1-yl)amino)-9H-purin-9-yl)-2-cyclopentène-1-méthanol;
le (±)cis-4-(2-amino-6-((8-aminooctyl)amino)9H-purin-9-yl)2-cyclopentène-1-méthanol; et
le (±)cis-4-(2-amino-6-((cyclopropylméthyl)amino)9H-purin-9-yl)-2-cyclopentène-1-méthanol.

**2.** Composé suivant la revendication 1, destiné à être utilisé en thérapie médicale.

**3.** Utilisation d'un composé suivant la revendication 1 ou d'un sel, ester ou sel d'un tel ester de celui-ci dans la fabrication d'un médicament pour le traitement ou la prophylaxie d'une infection virale.

**4.** Utilisation d'un composé suivant la revendication 1 ou d'un sel, ester ou sel d'un tel ester de celui-ci dans la fabrication d'un médicament pour le traitement ou la prophylaxie d'une infection rétrovirale ou virale par l'hépatite B.

**5.** Utilisation d'un composé suivant la revendication 1 ou d'un sel, ester ou sel d'un tel ester de celui-ci dans la fabrication d'un médicament pour le traitement ou la prophylaxie d'une infection par le VIH.

**6.** Formulation pharmaceutique comprenant un composé suivant la revendication 1 ou un sel pharmaceutiquement acceptable de celui-ci, et un support pharmaceutiquement acceptable.

**7.** Procédé de préparation de composés de formule (I):

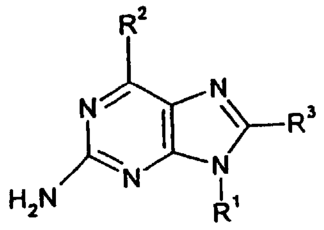

(I)

dans laquelle $R^1$ représente:

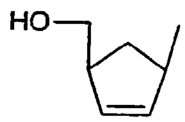

(A)

$R^2$ représente un groupe (2-cyclohexén-1-yl)thio, (2-cyclopentén-1-yl)amino, (8-aminooctyl)amino ou (cyclopropylméthyl)amino; et $R^3$ est de l'hydrogène,

qui comprend soit

A) le traitement d'un composé de formule (II) :

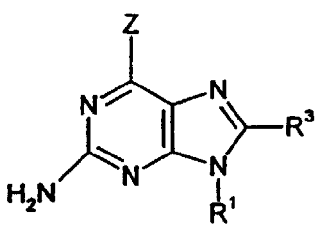

(II)

dans laquelle $R^1$ et $R^3$ sont tels que définis précédemment et Z représente un groupe précurseur pour le groupe $R^2$ précité avec un agent ou sous des conditions servant à convertir le groupe Z précurseur en le groupe $R^2$ désiré, ou bien Z représente un groupe thio sur lequel peut être substitué un groupe approprié pour former un composé de formule (I) dans laquelle $R^2$ est un groupe (2-cyclohexén-1-yl)thio, soit

B) la réaction d'un composé de formule :

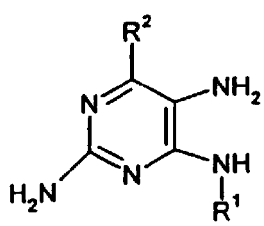

(dans laquelle $R^1$ et $R^2$ sont tels que définis précédemment) avec un agent servant à effectuer la formation du cycle imidazole dans le composé de formule (I) désiré; ou bien

lorsqu'un dérivé pharmaceutiquement acceptable d'un composé de formule (I) est formé, la conversion dudit dérivé en un composé de formule (I).

**Revendications pour l'Etat contractant suivant : ES**

1. Procédé de préparation de composés de formule (I):

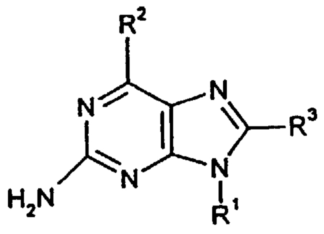

(I)

dans laquelle $R^1$ représente :

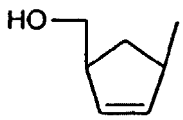

(A)

$R^2$ représente un groupe (2-cyclohexén-1-yl)thio, (2-cyclopentén-1-yl)amino, (8-aminooctyl)amino ou (cyclopropylméthyl)amino; et $R^3$ est de l'hydrogène,
qui comprend soit

A) le traitement d'un composé de formule (II) :

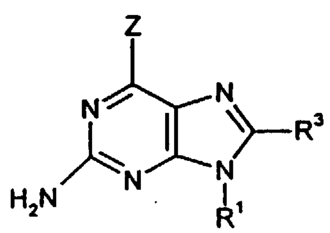

(II)

dans laquelle $R^1$ et $R^3$ sont tels que définis précédemment et Z représente un groupe précurseur pour le groupe $R^2$ précité avec un agent ou sous des conditions servant à convertir le groupe Z précurseur en le groupe $R^2$ désiré, ou bien Z représente un groupe thio sur lequel peut être substitué un groupe approprié pour former un composé de formule (I) dans laquelle $R^2$ est un groupe (2-cyclohexén-1-yl)thio, soit
B) la réaction d'un composé de formule :

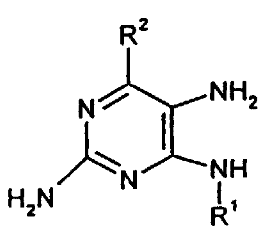

(dans laquelle $R^1$ et $R^2$ sont tels que définis précédemment) avec un agent servant à effectuer la formation du cycle imidazole dans le composé de formule (I) désiré; ou bien

lorsqu'un dérivé pharmaceutiquement acceptable d'un composé de formule (I) est formé, la conversion dudit dérivé en un composé de formule (I).

2. Composé choisi parmi :
   le (±)cis-4-(2-amino-6-(2-cyclohexén-1-yl)thio)-9H-purin-9-yl)-2-cyclopentène-1-méthanol;
   le (±)cis-4-(2-amino-6-(2-cyclohexén-1-yl)amino)9H-purin-9-yl)-2-cyclopentène-1-méthanol;
   le (±)cis-4-(2-amino-6-((8-aminooctyl)amino)-9H-purin-9-yl)-2-cyclopentène-1-méthanol; et
   le (±)-cis-4-(2-amino-6-((cyclopropylméthyl)amino)-9H-purin-9-yl)-2-cyclopentène-1-méthanol.

3. Composé suivant la revendication 2, destiné à être utilisé en thérapie médicale.

4. Utilisation d'un composé suivant la revendication 2 ou d'un sel, ester ou sel d'un tel ester de celui-ci dans la fabrication d'un médicament pour le traitement ou la prophylaxie d'une infection virale.

5. Utilisation d'un composé suivant la revendication 2 ou d'un sel, ester ou sel d'un tel ester de celui-ci dans la fabrication d'un médicament pour le traitement ou la prophylaxie d'une infection rétrovirale ou virale par l'hépatite B.

6. Utilisation d'un composé suivant la revendication 2 ou d'un sel, ester ou sel d'un tel ester de celui-ci dans la fabrication d'un médicament pour le traitement ou la prophylaxie d'une infection par le VIH.

7. Formulation pharmaceutique comprenant un composé suivant la revendication 2 ou un sel pharmaceutiquement acceptable de celui-ci, et un support pharmaceutiquement acceptable.